# EUROPEAN PATENT SPECIFICATION

(11) **EP 3 080 354 B1**
(45) Date of publication and mention of the grant of the patent: **07.08.2019**
(21) Application number: 14869474.8
(22) Date of filing: 12.12.2014
(51) Int. Cl.: D21H 27/30, B32B 29/00, D21H 11/02, D21H 11/08, D21H 27/38

(54) **MULTIPLY PAPERBOARD**
MEHRLAGIGE PAPPE
CARTON MULTICOUCHE

(30) Priority: 13.12.2013 US 201361915953 P
(43) Date of publication of application: 19.10.2016
(73) Proprietor: Stora Enso Oyj, 00101 Helsinki (FI)
(72) Inventor: PENG, Frank, S-663 41 Hammarö (SE); MOBERG, Anders, S-663 41 Hammarö (SE)
(74) Representative: Steinrud, Henrik
(86) International application number: PCT/IB2014/066851
(87) International publication number: WO 2015/087293

(56) References cited:
- WO-A1-00/14333
- WO-A1-95/34711
- WO-A1-97/11227
- WO-A1-2006/084883
- WO-A1-2006/084883
- WO-A1-2008/076056
- WO-A1-2012/039668
- WO-A1-2015/036930
- WO-A1-2015/036932
- US-A- 5 169 496
- US-A1- 2006 213 630
- VESTERLIND, E; ET AL.: 'Chemithermomechanical pulp made from birch at high temperature' NORDIC PULP AND PAPER RESEARCH JOURNAL vol. 21, no. 2, 2006, pages 216 - 221, XP055349252

## Description

### Technical field

A high-quality, multi-ply paperboard with high bulk and maintained strength.

### Background of the invention

There are a large number of applications for the use of paperboard. All these applications have their own specific requirements on the paperboard, thus the properties of the paperboard must differ depending on the intended end use.

Paperboard which is to be converted (e.g. coated, printed, cut, creased and folded) to packages on fast running automatic machines must have the required strength to withstand the strain and stress created during the converting. The Paperboard must also have a high bending resistance, not only to achieve good runnability during converting, but also crucial for the required package performance. For long shelf-life products the package must also have good barrier properties against light, oxygen and moisture. Also properties such as flatness and dimensional stability are important during converting.

The bulk of paperboard (inverse of density) is an important property since it contributes to the thickness of the paperboard. Increased, thickness improves the bending stiffness of the board. High bulk further enables the papermaker to reduce the amount of fibers in the paperboard which involves cost savings. However, a high bulk usually results in a decrease in internal strength. One challenge for the papermaker is to increase the bulk of the paperboard while maintaining the strength.

A paperboard generally comprises 1-5 plies (layers). A paperboard that is to be converted usually comprises more than one ply. A multi-ply paperboard usually shows higher bending resistance index than a single-ply paperboard. A paperboard which consists of three or more plies comprises top and back plies and one or more middle plies.

Chemical pulp is often times used in the top and back plies of the board to provide the board with strength and good printing properties. The middle ply of the board may contain both mechanical pulp and/or chemical pulp. Mechanical pulp, such as CTMP, is a desirable raw material since it has rather low comparatively density and can be produced to a lower cost than chemical pulp. Also, mechanical pulp has a higher yield and thus a higher efficiency of raw material usage. In high quality boards, softwood CTMP is the most common mechanical pulp used for the middle ply because softwood CTMP, in addition to high bulk, also has long fibers that can provide good internal bonding. Chemical pulp is normally also used in the middle ply in combination with mechanical pulp, as reinforcement, due to its high strength properties.

Patent specification WO2006084883 discloses a high quality paperboard with a middle ply comprising a high amount of hardwood CTMP. However, there is an upper limit to how high bulk one can achieve at maintained strength at the production of the paperboard disclosed in WO2006084883. Thus, there still remains a need for a high quality paperboard with a high bulk and maintained strength.

### Summary of the invention

The present invention discloses a high quality paperboard comprising at least three plies; a surface ply, a middle ply and a back ply, wherein the middle ply comprises at least 50 %, preferably at least 80%, more preferably at least 90% of high - temperature chemithermomechanical pulp (HT-CTMP) from hardwood fibers, calculated on the total fiber weight of said middle ply.

It has surprisingly been shown that the use of HT-CTMP from hardwood fibers in the middle ply enables the production of a paperboard with a very high bulk and with maintained strength properties. Moreover, the use of HT-CTMP from hardwood fibers significantly improves the brightness properties and the formation of the paperboard. The middle ply of the paperboard exhibits a density of below 400 kg/m3, preferably of below 350 kg/m3, even more preferably of below 300 kg/m3, a z-strength of at least 150 kPa, preferably of at least 250 kPa, most preferably of at least 350 kPa and a Scott Bond value of at least 100 J/m2, preferably of at least 150 J/m2.

The hardwood pulp in said middle pulp has a density (pulp sheet density, ISO 534:2005) of below 300 kg/m3, preferably of below 250 kg/m3 and even more preferably 200 kg/m3.

At least one of the plies, preferably the middle ply, of the inventive paperboard may comprise dry strength additives. The strength additives may be selected from the group consisting of cationic starch, anionic polymers, nanofibrillated cellulose, polyvinylamine, chitosan, primary and secondary amines, polyethylene amines and modified polyacrylamides and combinations thereof. The anionic polymers may, e.g., be carboxymethyl cellulose (CMC) and/or anionic polyacrylamide (A-PAM). The middle ply can comprise A-PAM in an amount of at least 0,1 kg/ton, preferably of at least 0,2 kg/ton. addition of such dry strength additives enhances the strength properties of the paperboard and enables the use a high amount of HT CTMP from hardwood fibers in the middle ply. In one preferred embodiment, at least one ply comprises at least one cationic polymer, such as cationic starch, and at least one anionic polymer, such as anionic CMC (carboxymethyl cellulose), or anionic polyacrylamide (A-PAM). Said cationic and anionic polymers may be added to the furnish forming said ply in consecutive steps. This improves the strength of the resulting board product. Alternatively, said polymers may be added simultaneously or pre-mixed to the furnish mixed.

The hardwood pulp used in accordance with the invention can, e.g., be from eucalyptus, aspen, acacia, maple, beech, poplar and/or birch.

### Detailed description of the invention

The paperboard of the current invention is a high quality paperboard suitable for use as, e.g., a liquid packaging paperboard, food service board, graphical paperboard or cigarette paperboard.

A high quality paperboard is a paperboard with high strength, in order to be able to withstand converting, good protective properties as well as high appearance.

Preferably, the multi-ply paperboard of the invention comprises three plies; a top ply, a back ply and at least one middle ply. The paperboard may comprise further plies, arranged between said outer plies and said middle ply. These intermediate plies may have the same or different fiber composition as the middle ply. The paperboard may for example comprise four or five plies in total.

In accordance with the invention, the middle ply comprises at least 50 %, preferably at least 80%, and more preferably at least 90%, of hardwood HT-CTMP, which provides the paperboard with high bulk without any substantial decrease in strength of the final paperboard product. The middle ply may further comprise a small amount of bleached or unbleached chemical pulp, e.g. 10%, 15 or 20% by weight as calculated on the total fiber weight of said middle ply. The top ply preferably comprises hardwood and/or softwood chemical pulp, which gives the product good strength and printing properties. The back ply may comprise hardwood and/or softwood chemical pulp.

The term "high -temperature chemithermomechanical pulp (HT-CTMP)" as used herein refers to pulp that has been pre-heated to a temperature of at least 140 °C, preferably of at least 150 °C, or even more preferably of at least 160 °C prior to the refining step.

Surprisingly it has been shown that it is possible to produce a paperboard comprising such high amount of HT-CTPMP from hardwood fibers in a middle ply and still maintain high strength.

The inventive paperboard may also comprise dry strength additives. The dry strength additives that may be used include, but are not limited to; starch, polyacrylamide, carboxymethyl cellulose (CMC) nanofibrillated polysaccharide. Nanofibrillated polysaccharide shall in this context include bacterial cellulose or nanocellulose spun with either traditional spinning techniques or with electrostatic spinning. Also microfibrillated cellulose (MFC), also known as nanocellulose, cellulose whiskers, microcrystalline cellulose (MCC), nanocrystalline cellulose (NCC) or regenerated cellulose fibers and particles are herein included in the definition of nanofibrillated polyschharide.

In one embodiment, one of the plies, preferably the middle ply, comprises cationic starch and anionic CMC. Cationic starch and anionic CMC may be added to one of the plies, preferably the middle ply, in consecutive step. This may e.g. be accomplished in accordance with the methods disclosed in WO2006120235 or WO2006041401. This has been shown to increase the strength properties of the resultant paperboard product even further. Preferably, the middle ply comprises cationic starch to an amount of at least 20 kg/ton, or preferably at least 30 kg/ton, and CMC to an amount of at least 1 kg/ton or at least 2 kg/ton.

### Measurement and evaluation methods

The following methods and standards apply both to the definitions of the appended claims and to the measurements performed in the example below.

Tensile Index: ISO 1924-3:2005
Scott Bond: TAPPI UM-403
Z-strength: SCAN-P 80:98
Density (paperboards, sample 1 - 2, table 4): ISO 534:2011
Drainage resistance: SCAN M3:65
Strain at failure: ISO 1924-3:2005
Tensile energy abs. index: ISO 1924-3:2005
Tensile stiffness index ISO 1924-3:2005
Density (pulp sheet density, table 1): ISO 534:2005

### Example

In order to evaluate the high quality paperboard of the invention, a test series was performed in which a centerply of a paperboard, i.e. a ply to be used as a middle ply in a multilayer paperboard, in accordance with the invention was compared with a conventional centerply of a paperboard.

Strength properties of the centerplies were investigated. All tests were performed according to the methods and standards as indicated above and all analyses were carried out according to available standards after conditioning at 23 C, 50%RH.

### Centerply Samples

Centerply samples in accordance with the invention and reference centerply samples based on conventional spruce CTMP were produced using a Dymanic Sheet Former (Formette Dynamique). The properties of the pulps used in the centerply samples are shown in table 1. Comparative tests were performed on conventional centerplies (sample 1 - 3) and the centerplies in accordance with the invention (Sample 4 - 6). The physical properties of the centerplies are shown in Table 2.

**Table 1**

| | **BSKP** | **CTMP spruce** | **HT-CTMP Birch** |
|---|---|---|---|
| Drainage resistance (°SR) | 30,7 | | |
| Canadian Standard Freeness (ml) | | 537 | 712 |
| Density (kg/m3) | 748 | 293 | 218 |
| Tensile index (Nm/g) | 78,0 | 25,1 | Too weak to measure |
| Strain at break (%) | 4,00 | 1,62 | Too weak to measure |
| Tensile energy abs. index (J/g) | 2,11 | 0,28 | Too weak to measure |
| Tensile stiffness index (kNm/g) | 7,35 | 2,94 | Too weak to measure |

| | | | |
|---|---|---|---|
| BSKP = Bleached Softwood Kraft Pulp of spruce/pine SR° 30 CTMP = Chemi-thermomechanical pulp of spruce CSF 537ml. HT-CTMP = Chemi-thermomechanical pulp of Birch that has been pre-heated to a temperature of 170°C prior to the refining step, CSF 712ml | | | |

**Table 2**

| **Sample** | **1** | **2** | **3** | **4** | **5** | **6** |
|---|---|---|---|---|---|---|
| CTMP Spruce (%) | 80 | 80 | 80 | | | |
| HT CTMP Birch (%) | | | | 80 | 80 | 80 |
| BSKP (%) | 20 | 20 | 20 | 20 | 20 | 20 |
| Starch (kg/t) | 0 | 20 | 40 | 0 | 20 | 40 |
| CMC (kg/t) | 0 | 2 | 4 | 0 | 2 | 4 |
| Grammage (g/m2) | 149,4 | 152,9 | 153,3 | 147,4 | 149,5 | 155,9 |
| Density (kg/m3) | 395,2 | 397,4 | 411,5 | 330,8 | 346,8 | 351,8 |
| Tensile index GM (Nm/g) | 45,2 | 54,4 | 64,1 | 19,9 | 31,3 | 41,2 |
| Tensile index MD (Nm/g) | 68,8 | 85,0 | 95,2 | 31,3 | 53,4 | 69,9 |
| Tensile index CD (Nm/g) | 29,7 | 34,9 | 43,2 | 12,6 | 18,3 | 24,3 |
| Strech MD (%) | 2,3 | 2,8 | 2,8 | 1,1 | 1,6 | 2,0 |
| Strech CD (%) | 2,6 | 3,1 | 3,4 | 1,6 | 2,3 | 2,7 |
| Tensile absorption index GM (J/g) | 0,8 | 1,1 | 1,3 | 0,2 | 0,4 | 0,7 |
| Tensile absorption index MD (J/g) | 1,1 | 1,6 | 1,8 | 0,2 | 0,6 | 0,9 |
| Tensile absorption index CD (J/g) | 0,5 | 0,8 | 1,0 | 0,1 | 0,3 | 0,5 |
| Tensile stiffness index GM (kNm/g) | 4,4 | 4,5 | 5,0 | 3,0 | 3,6 | 4,0 |
| Tensile stiffness index MD (kNm/g) | 6,9 | 7,1 | 7,6 | 5,6 | 7,2 | 7,6 |
| Tensile stiffness index CD (kNm/g) | 2,8 | 2,8 | 3,3 | 1,6 | 1,8 | 2,1 |
| Scott-Bond (J/m²) | 127,6 | 186,2 | 210,0 | 79,4 | 141,0 | 197,6 |
| Z-strength (kPa) | 163,7 | 218,7 | 270,6 | 73,4 | 152,8 | 232,5 |

The comparison shows, surprisingly, that the strength properties of a centerply comprising a high amount of HT-CTMP are comparable to the strength properties of a conventional centerply comprising CTMP from softwood, especially when strength additives are added.

## Claims

1. A high quality paperboard comprising at least three plies; a surface ply, a middle ply and a back ply, wherein the middle ply comprises at least 50 %, preferably at least 80%, more preferably at least 90%, by weight of high - temperature chemithermomechanical pulp (HT-CTMP) from hardwood fibers, all percentages calculated on the total fiber weight of said middle ply and wherein the middle ply exhibits a density according to ISO 534:2011 of below 400 kg/m3, a z-strength according to SCAN-P 80:98 of at least 150 kPa and a Scott Bond value according to TAPPI UM-403 of at least 100 J/m2.

2. A paperboard according to claim 1, wherein the middle ply exhibits a density according to ISO 534:2011 of below 350 kg/m3, preferably of below 300 kg/m3 and a z-strength according to SCAN-P 80:98 of at least 250 kPa, preferably of at least 350 kPa.

3. A paperboard according to any one of claims 1 - 2, wherein the middle ply exhibits a Scott Bond value according to TAPPI UM-403 of at least 150 J/m2.

4. A paperboard according to any one of claims 1 - 3, wherein said hardwood pulp in said middle pulp has a density according to ISO 534:2005 of below 300 kg/m3, preferably of below 250 kg/m3 and even more preferably 200 kg/m3.

5. A paperboard according to any one of claims 1 - 4, wherein the middle ply comprises dry strength additives.

6. A paperboard according to claim 5, wherein said dry strength additives are selected from the group consisting of cationic starch, anionic polymers, nanofibrillated cellulose, polyvinylamine, chitosan, primary and secondary amines, polyethylene amines, modified polyacrylamides and combinations thereof.

7. A paperboard according to any one of claims 1 - 6, wherein the middle ply comprises cationic starch in an amount of at least 20 kg/ton, preferably at least 40 kg/ton, and/or anionic polymer in an amount of at least 0,1 kg/ton.

8. A paperboard according to any one of claims 1 - 6, wherein the middle ply comprises CMC in an amount of at least 1 kg/ton, preferably of at least 1,5 kg/ton.

9. A paperboard according to any one of claims 1 - 6, wherein the middle ply comprises A-PAM in an amount of at least 0,1 kg/ton, preferably of at least 0,2 kg/ton.

10. A paperboard according to any one of claims 1 - 9, wherein the hardwood fibers are from birch and/or eucalyptus

11. A paperboard for holding liquids **characterized in that** it is produced from the paperboard of any of the claims 1 - 10.

12. A paperboard for holding food **characterized in that** it is produced from the paperboard of any of the claims 1 - 10.

## Patentansprüche

1. Karton hoher Qualität, umfassend mindestens drei Lagen; eine Oberflächenlage, eine mittlere Lage und eine hintere Lage, wobei die mittlere Lage nach Gewicht mindestens 50 %, vorzugsweise mindestens 80 %, noch bevorzugter mindestens 90 %, chemithermomechanischen Hochtemperatur-Zellstoff (HT-CTMP) aus Hartholzfasern umfasst, wobei alle prozentualen Anteile basierend auf dem Fasergesamtgewicht der mittleren Lage berechnet werden, und wobei die mittlere Lage eine Dichte gemäß ISO 534:2011 von weniger als 400 kg/m3, eine z-Stärke gemäß SCAN-P 80:98 von mindestens 150 kPa und einen Scott-Bond-Wert gemäß TAPPI UM-403 von mindestens 100 J/m2 aufweist.

2. Karton nach Anspruch 1, wobei die mittlere Lage eine Dichte gemäß ISO 534:2011 von weniger als 350 kg/m3, vorzugsweise von weniger als 300 kg/m3, und eine z-Festigkeit gemäß SCAN-P 80:98 von mindestens 250 kPa, vorzugsweise von mindestens 350 kPa, aufweist.

3. Karton nach einem der Ansprüche 1-2, wobei die mittlere Lage einen Scott-Bond-Wert gemäß TAPPI UM-403 von mindestens 150 J/m2 aufweist.

4. Karton nach einem der Ansprüche 1-3, wobei der Hartholzzellstoff in der mittleren Lage eine Dichte gemäß ISO 534:2005 von weniger als 300 kg/m3, vorzugsweise von weniger als 250 kg/m3, und sogar noch bevorzugter von 200 kg/m3 aufweist.

5. Karton nach einem der Ansprüche 1-4, wobei die mittlere Lage Trockenfestigkeitsadditive umfasst.

6. Karton nach Anspruch 5, wobei die Trockenfestigkeitsadditive ausgewählt sind aus der Gruppe, bestehend aus kationischer Stärke, anionischen Polymeren, nanofibrillierter Cellulose, Polyvinylamin, Chitosan, primären und sekundären Aminen, Polyethylenaminen, modifizierten Polyacrylamiden und Kombinationen davon.

7. Karton nach einem der Ansprüche 1-6, wobei die mittlere Lage kationische Stärke in einer Menge von mindestens 20 kg/t, vorzugsweise mindestens 40 kg/t, und/oder anionisches Polymer in einer Menge von mindestens 0,1 kg/t umfasst.

8. Karton nach einem der Ansprüche 1-6, wobei die mittlere Lage CMC in einer Menge von mindestens 1 kg/t, vorzugsweise von mindestens 1,5 kg/t umfasst.

9. Karton nach einem der Ansprüche 1-6, wobei die mittlere Lage A-PAM in einer Menge von mindestens 0,1 kg/t, vorzugsweise von mindestens 0,2 kg/t umfasst.

10. Karton nach einem der Ansprüche 1-9, wobei die Hartholzfasern aus Birke und/oder Eukalyptus bestehen.

11. Karton zum Fassen von Flüssigkeiten, **dadurch gekennzeichnet, dass** er aus dem Karton nach einem der Ansprüche 1-10 hergestellt ist.

12. Karton zum Fassen von Nahrungsmitteln, **dadurch gekennzeichnet, dass** er aus dem Karton nach einem der Ansprüche 1-10 hergestellt ist.

## Revendications

1. Carton de haute qualité comprenant au moins trois couches ; une couche de surface, une couche intermédiaire et une couche arrière, dans lequel la couche intermédiaire comprend au moins 50 %, de préférence au moins 80 %, plus préférablement au moins 90 %, en poids de pâte chimicothermomécanique à haute température (HT-CTMP) en fibres de bois dur, tous pourcentages calculés sur le poids total en fibres de ladite couche intermédiaire et dans lequel la couche intermédiaire présente une densité selon la norme ISO 534:2011 inférieure à 400 kg/m3, une résistance z selon la norme SCAN-P 80:98 d'au moins 150 kPa et une valeur Scott Bond selon la norme TAPPI UM-403 d'au moins 100 J/m2.

2. Carton selon la revendication 1, dans lequel la couche intermédiaire présente une densité selon la norme ISO 534:2011 inférieure à 350 kg/m3, de préférence inférieure à 300 kg/m3 et une résistance z selon la norme SCAN-P 80:98 d'au moins 250 Kpa, de préférence d'au moins 350 Kpa.

3. Carton selon l'une quelconque des revendications 1 à 2, dans lequel la couche intermédiaire présente une valeur de Scott Bond selon la norme TAPPI UM-403 d'au moins 150 J/m2.

4. Carton selon l'une quelconque des revendications 1 à 3, dans lequel ladite pâte de bois dur dans ladite pâte intermédiaire a une densité selon la norme ISO 534:2005 inférieure à 300 kg/m3, de préférence inférieure à 250 kg/m3 et encore plus préférablement à 200 kg/m3.

5. Carton selon l'une quelconque des revendications 1 à 4, dans lequel la couche intermédiaire comprend des additifs de résistance à sec.

6. Carton selon la revendication 5, dans lequel lesdits additifs de résistance à sec sont sélectionnés parmi le groupe constitué de l'amidon cationique, des polymères anioniques, de la cellulose nanofibrillée, de la polyvinylamine, du chitosan, des amines primaires et secondaires, des amines en polyéthylène, des polyacrylamides modifiés et des combinaisons de ceux-ci.

7. Carton selon l'une quelconque des revendications 1 à 6, dans lequel la couche intermédiaire comprend l'amidon cationique d'une quantité d'au moins 20 kg/tonne, de préférence au moins 40 kg/tonne, et/ou du polymère anionique en une quantité d'au moins 0,1 kg/tonne.

8. Carton selon l'une quelconque des revendications 1 à 6, dans lequel la couche intermédiaire comprend du CMC en une quantité d'au moins 1 kg/tonne, de préférence d'au moins 1,5 kg/tonne.

9. Carton selon l'une quelconque des revendications 1 à 6, dans lequel la couche intermédiaire comprend de l'A-PAM en une quantité d'au moins 0,1 kg/tonne, de préférence d'au moins 0,2 kg/tonne.

10. Carton selon l'une quelconque des revendications 1 à 9, dans lequel les fibres en bois dur proviennent du bouleau et/ou de l'eucalyptus

11. Carton destiné à contenir des liquides, **caractérisé en ce qu'**il est produit à partir du carton selon l'une quelconque des revendications 1 à 10.

12. Carton destiné à contenir des aliments, **caractérisé en ce qu'**il est produit à partir du carton selon l'une quelconque des revendications 1 à 10.
